# EUROPÄISCHE PATENTSCHRIFT

(11) **EP 1 783 225 B1**
(45) Veröffentlichungstag und Bekanntmachung des Hinweises auf die Patenterteilung: **15.09.2010**
(21) Anmeldenummer: 06018532.9
(22) Anmeldetag: 21.06.1999
(51) Int. Cl.: C12N 15/35, C12N 15/62, C07K 14/015, C12N 5/10, A61K 39/23, A61K 48/00

(54) **Strukturprotein von AAV, seine Herstellung und Verwendung**
AAV structural protein, production and use thereof
Protéine structurale de Virus Associé aux Adenovirus, sa production et son utilisation

(30) Priorität: 19.06.1998 DE 19827457
(43) Veröffentlichungstag der Anmeldung: 09.05.2007
(62) Teilanmeldung aus: 99929292.3
(73) Patentinhaber: MediGene AG, 82152 Planegg/Martinsried (DE)
(72) Erfinder: Hallek, Michael, 50935 Köln (DE); Ried, Martin, 82319 Starnberg (DE); Deleage, Gilbert, 69008 Lyon (FR); Girod, Anne, 82287 Jesenwang (DE)
(74) Vertreter: Bösl, Raphael Konrad

(56) Entgegenhaltungen:
- WO-A-96/00587
- WO-A-97/38723
- WO-A-99/67393
- OHNO K. ET AL.: "Cell-specific targeting of Sindbis virus vectors displaying IgG-binding domains of protein A." NATURE BIOTECHNOLOGY, Bd. 15, August 1997 (1997-08), Seiten 763-767, XP002128038
- WICKHAM T. J. ET AL.: "Adenovirus targeted to heparan-containing receptors increases its gene delivery efficiency to multiple cell types." NATURE BIOTECHNOLOGY, Bd. 14, November 1996 (1996-11), Seiten 1570-1573, XP002128039
- YANG Q. ET AL.: "Development of novel cell surface CD34-targeted recombinant adenoassociated virus vectors for gene therapy." HUMAN GENE THERAPY, Bd. 9, September 1998 (1998-09), Seiten 1929-1937, XP000867311
- GIROD A. ET AL.: "GENETIC CAPSID MODIFICATIONS ALLOW EFFICIENT RE-TARGETING OF ADENO-ASSOCIATED VIRUS TYPE 2" NATURE MEDICINE, Bd. 5, Nr. 9, 1999, Seiten 1052-1056, XP002128040

### SEQUENCE LISTING

<110> MediGene AG
<120> Strukturprotein von AAV, seine Herstellung und Verwendung
<130> M26890PCEPT1
<160> 8
<170> Patentin version 3.3
<210> 1
   <211> 14
   <212> PRT
   <213> Artificial
<220>
   <223> inneres Fragment
<400> 1 Gln Ala Gly Thr Phe Ala Leu Arg Gly Asp Asn Pro Gln Gly 1 5 10
<210> 2
   <211> 10
   <212> PRT
   <213> Artificial
<220>
   <223> inneres Fragment
<400> 2
   Tyr Lys Gln Ile Ser Ser Gln Ser Gly Ala
   1               5                   10
<210> 3
   <211> 10
   <212> PRT
   <213> Artificial
<220>
   <223> inneres Fragment
<400> 3
   Tyr Leu Thr Leu Asn Asn Gly Ser Gln Ala
   1               5                   10
<210> 4
   <211> 10
   <212> PRT
   <213> Artificial
<220>
   <223> inneres Fragment
<400> 4
   Tyr Tyr Leu Ser Arg Thr Asn Thr Pro Ser
   1               5                   10
<210> 5
   <211> 10
   <212> PRT
   <213> Artificial
<220>
   <223> inneres Fragment
<400> 5
   Glu Glu Lys Phe Phe Pro Gln Ser Gly Val
   1               5                   10
<210> 6
   <211> 10
   <212> PRT
   <213> Artificial
<220>
   <223> inneres Fragment
<400> 6
   Asn Pro Val Ala Thr Glu Gln Tyr Gly Ser
   1               5                   10
<210> 7
   <211> 10
   <212> PRT
   <213> Artificial
<220>
   <223> inneres Fragment
<400> 7
   Leu Gln Arg Gly Asn Arg Gln Ala Ala Thr
   1               5                   10
<210> 8
   <211> 10
   <212> PRT
   <213> Artificial
<220>
   <223> inneres Fragment
<400> 8
   Asn Val Asp Phe Thr Val Asp Thr Asn Gly
   1               5                   10

## Beschreibung

Die vorliegende Erfindung betrifft ein Strukturprotein von Adeno-assoziiertem Virus (AAV), das mindestens eine Mutation enthält, die eine Erhöhung der Infektiosität bewirkt.

Das AAV-Virus gehört zur Familie der Parvoviren. Diese zeichnen sich durch ein ikosaedrisches, unbehülltes Kapsid mit einem Durchmesser von 18 bis 30 nm aus, welches eine lineare, einzelstrangige DNA von ca. 5 kb enthält. Für eine effiziente Vermehrung von AAV ist eine Coinfektion der Wirtszelle mit Helferviren, beispielsweise mit Adenoviren, Herpesviren oder Vacciniaviren erforderlich. In Abwesenheit eines Helfervirus geht AAV in einen Latenzzustand über, wobei das Virusgenom in der Lage ist, stabil in das Wirtszellgenom zu integrieren. Die Eigenschaft von AAV, in das Wirtsgenom zu integrieren, macht es als Transduktionsvektor für Säugetierzellen besonders interessant. Für die Vektorfunktionen genügen im allgemeinen die beiden ca. 145 bp langen invertierten terminalen Wiederholungssequenzen (ITR: "Inverted Terminal Repeats"). Sie tragen die "cis" notwendigen Signale für Replikation, Verpackung und Integration in das Wirtszellgenom. Zur Verpackung in rekombinante Vektorpartikel wird ein Vektorplasmid, welches die Gene für nicht-strukturelle Proteine (Rep-Proteine) und für strukturelle Proteine (Cap-Proteine) trägt, in verpackungsgeeignete Zellen, z.B. HeLa- oder 293-Zellen, transfiziert, die hierauf beispielsweise mit Adenovirus infiziert werden. Nach einigen Tagen erhält man ein Lysat, welches rekombinante AAV-Partikel enthält.

Das AAV-Kapsid besteht aus drei verschiedenen Proteinen: VP1, VP2 und VP3, deren relative Anteile 5% VP1, 5% VP2 und 90% VP3 sind. Die AAV-Kapsidgene sind am rechten Ende des AAV-Genoms lokalisiert und werden durch überlappende Sequenzen desselben offenen Leserahmens (ORF) unter Verwendung verschiedener Startkodons kodiert. Das VP1-Gen enthält die ganze VP2-Gensequenz, welche wiederum die ganze VP3-Gensequenz mit einem spezifischen N-terminalen Bereich enthält. Die Tatsache, daß die überlappenden Leserahmen für alle drei AAV-Kapsid-Proteine kodieren, ist für die obligatorische Expression aller Kapsid-Proteine, wenn auch zu unterschiedlichen Anteilen, verantwortlich.

Die Molekularmassen der Kapsid-Proteine sind 87 kD für VP1, 73 kD für VP2 und 62 kD für VP3. Die Sequenzen der Kapsidgene sind in Srivastava, A. et al. (1983), J. Virol., 45, 555-564; Muzyczka, N. (1992), Curr. Top. Micro. Immunol., 158, 97-129, Ruffing, N. et al. (1992), J. Virol., 66, 6922-6930 oder Rutledge, E. A. et al. (1998) J. Virol. 72, 309-319 beispielsweise beschrieben. Die physikalische und genetische Karte des AAV-Genoms ist beispielsweise bei Kotin, R. M. (1994), Human Gene Therapy, 5, 793-801 beschrieben.

Zudem sind verschiedene AAV-Serotypen bekannt, von denen der menschliche AAV-Serotyp 2 (AAV2) ein Virusvektor mit vorteilhaften Eigenschaften für die somatische Gentherapie darstellt. Die wesentlichen Vorteile sind die fehlende Pathogenität für den Menschen, die stabile Integration viraler DNA in das zelluläre Genom, die Fähigkeit, nicht teilende Zellen zu infizieren, die Stabilität des Virions, was die Aufreinigung zu hohen Titern (10" Partikel pro ml) ermöglicht, die geringe Immunogenität sowie das weitgehende Fehlen viraler Gene und Genprodukte im rekombinanten AAV-Vektor, was unter Sicherheitsaspekten für die Verwendung in der Gentherapie vorteilhaft ist. Die Klonierung von Genen in den AAV-Vektor erfolgt mittlerweile nach den dem Fachmann allgemein bekannten Methoden, wie sie z.B. in WO 95/ 23 867, bei Chiorini, J. A. et al. (1995), Human Gene Therapy, 6, 1531-1541 oder bei Kotin, R. M. (1994), supra, beschrieben sind.

Im allgemeinen hat beispielsweise AAV2 ein breites Wirkspektrum. Epitheliale Gewebe, wie menschliche epitheliale Tumorzellinien, aber auch primäres Tumormaterial, wie Zervix- oder Ovarialkarzinom oder Melanom, und menschliche Keratinozyten werden sehr effizient infiziert (70-80%), wohingegen hämatopoetische Zellen, wie lymphohämatopoetische Zellen mit 10- bis 100fach geringerer Effizienz (0,5-5%) infiziert werden (Mass et al. (1998) Human Gene Therapy, 9, 1049 - 1059). Eine Ursache hierfür könnte sein, daß für die Aufnahme von AAV in die Zelle eine Interaktion zwischen AAV und einem AAV-Rezeptor auf der Oberfläche der Zelle notwendig ist. So ist beispielsweise der putative, primäre AAV2-Rezeptor ein Zellmembran-Glykoprotein von 150 kD (Mizukami, H. et al. (1996), Virology, 217, 124-130) oder Heparan-Sulfat-Proteoglycan (Summerford, C. & Samulski, R. J. (1998), J. Virol., 72, 1438-1445). Als mögliche Sekundärrezeptoren wurden bestimmt: _{α}V_{β}5 Integrin (Summerford et al., (1999) Nature Medicine 5, 78-82) und human fibroblast growth factor receptor 1 (Qing et al., (1999) Nature Medicine 5, 71-77). Bindungsstudien zeigten nun, daß die Oberflächendichte dieses Rezeptors auf Zellen, die durch AAV2 nicht effizient infiziert werden, herabgesetzt ist.

Es ist nun bekannt, daß durch eine genetische Modifikation von Kapsid-Proteinen von Retroviren und Adenoviren Bindungsstellen für Rezeptoren in das Kapsid eingebracht werden können, die nur auf bestimmten Zellen exprimiert werden und somit ein Rezeptor-vermitteltes Targeting von Vektoren ermöglicht wurde (s. z.B. Cosset, F. L. & Russell, S. J. (1996), Gene Ther., 3, 946-956, Douglas, J. T. et al. (1996), Nat. Biotechnol., 14, 1574-1578, Krasnykh, V. N. et al. (1996), J. Virol., 70, 6839-6846, Stevenson, S. C. et al. (1997), J. Virol., 71, 4782-4790 oder Wickham, T. J. et al. (1996), Nat. Biotechnol., 14, 1570-1573). In WO 96/00587 wird auch auf AAV-Kapsid-Fusionsproteine hingewiesen, die heterologe Epitope klinisch relevanter Antigene enthalten sollen, wodurch eine Immunantwort induziert werden soll und die mit der Kapsidbildung nicht interferieren sollen. Die Veröffentlichung enthält jedoch nur einen allgemeinen Hinweis ohne nähere Angaben zur Ausführbarkeit, insbesondere zu geeigneten Insertionsstellen. Steinbach et al. (1997) (Biol. Abstr. 104, Ref. 46570) beschäftigten sich mit der in-vitro Assemblierung von AAV Partikeln, die zuvor im baculo-System exprimiert wurden. Es werden auch Mutationen am cap-Gen gemacht, die aber nicht zu einer Änderung des Tropismus, sondern zu einem Plasmidkonstrukt führen sollten, bei dem jeweils nur ein VP-Protein exprimiert wird. Eine Veränderung der Infektiosität wird nicht erwähnt. Ruffing et al. (1994) (J.Gen.Virol. 75, 3385-3392) beabsichtigten, den natürlichen Tropismus von AAV2 zu untersuchen. Zu diesem Zweck wurden Mutationen am C-Terminus des AAV2-VP-Proteins eingeführt, wobei von der (aufgrund falscher Ausgangsdaten irrigen) Annahme ausgegangen wurde, damit ein RGD-Motiv zu ändern. Die Mutation bewirkte lediglich eine reduzierte Infektiosität.

Ein indirektes Targeting ist aus Bartlett et al. (1999; Nat. Biotechnol. 17, 181-186) bekannt. Dabei wurde ein bi-spezifischer Antikörper verwendet, der sowohl gegen das AAV2-Kapsid als auch eine Zielzelle gerichtet war. Das Viruskapsid wurde jedoch weder kovalent verknüpft noch modifiziert oder ein Kapsid-Protein mutiert. Der bisher einzige Versuch eines direkten Targetings bei AAV2 wurde von Yang et al. (1998; Hum. Gene Ther. 1, 1929-1937) unternommen. Dabei wurden Single-Chain-Antikörperfragmente gegen das CD34-Molekül mit dem N-Terminus von VP2 fusioniert, direkt am N-Terminus von VP1 insertiert. Diese Methode weist jedoch 2 deutliche Nachteile auf. Zum einen war der Infektionstiter sehr niedrig und zum anderen mußte das Fusionsprotein für eine erfolgreiche Verpackung mit unmutierten Kapsidproteinen VP1, VP2 und VP3 koexprimiert werden. Daraus resultierte aber eine Mischung aus chimärischen und Wildtyp-Kapsidproteinen, deren Zusammensetzung und damit Wirksamkeit nicht vorhersehbar war. Im übrigen war auch die Verpackungseffizienz und die Infektiosität über den WildtypRezeptor auf HeLa-Zellen gegenüber dem Wildtyp erheblich verringert.

Aufgabe der vorliegenden Erfindung war es daher, AAV so zu modifizieren, daß ein spezifischerer und effizienterer Gentransfer möglich ist als mit bekannten AAV-Vektoren.

Überraschenderweise wurde nun gefunden, daß Struktur- bzw. Kapsid-Proteine von AAV so modifiziert werden können, daß dadurch eine Erhöhung der Infektiosität bewirkt wird.

Ein Gegenstand der vorliegenden Erfindung ist daher ein Strukturprotein von AAV, das mindestens eine Mutation enthält, die durch ein oder mehrere Insertionen bewirkt wird/werden, wobei jede der Insertionen in VP3 unmittelbar vor und/oder nach einer Aminosäure in der Sequenz ausgewählt aus YKQIS SQSGA, YLTLN NGSQA, YYLSR TNTPS, EEKFF PQSGV, NPVAT EQYGS, LQRGN RQAAT und NVDFT VDTNG lokalisiert ist. Vorzugsweise bewirkt die Mutation eine Erhöhung der Infektiosität. Durch die Erhöhung der Infektiosität kann beispielsweise ein spezifischer bzw. effizienter Gentransfer von wenig infiziertem Gewebe, wie z.B. hämatopoetischem Gewebe, erzielt werden. Unter Änderung und insbesondere Erhöhung im Sinne dieser Erfindung ist keine generelle sondern eine zellspezifische Änderung oder Erhöhung zu verstehen, also in Hinsicht auf einen bestimmten Zelltyp. Damit sind unter Erhöhung der Infektiosität auch Fälle erfaßt, in denen die Infektiosität für bestimmte Zellen erniedrigt und lediglich für einen anderen Zelltyp oder mehrere andere Zelltypen erhöht ist.

Vorzugsweise ist/sind die Mutation/en an der Virusoberfläche lokalisiert. Zur Bestimmung der Oberflächen-lokalisierten Bereiche der Strukturproteine wurde gemäß der vorliegenden Erfindung überraschenderweise gefunden, daß CPV- und AAV2-Sequenzen und -Strukturen vergleichbar sind. Man kann daher vorzugsweise auf bekannte Kristallstrukturen von Parvoviren wie von Parvovirus B19 oder von CPV (Canine-Parvovirus) zurückgreifen und mit Hilfe von Homologievergleichen Proteindomänen identifizieren, die für die AAV/AAV-Rezeptor-Interaktion wichtig sind und die modifiziert werden können. Gemäß der vorliegenden Erfindung hat daher beispielsweise ein computerunterstützter Vergleich zwischen CPV und AAV2 bzw. Parvovirus B 19 und AAV2 überraschenderweise reproduzierbar zur Identifikation von Schleifen (Loops) in VP3 geführt, deren Sequenz variiert, d.h. die eine geringe Homologie besitzen und die für den Tropismus bzw. die unterschiedliche Infektiosität des Virus verantwortlich sein können. So wurde die bekannte Kristallstruktur des CPV VP2-Kapsid-Proteins (z.B. Luo M.(1988), J. Mol. Biol., 200, 209-211; Wu und Rossmann (1993), J.Mol.Biol., 233, 231-244) aufgrund der hohen Ähnlichkeit zum AAV2 VP3 in der sekundären Struktur des Proteins als Muster genommen, um die Regionen herauszufinden, die auf der viralen Kapsidoberfläche exponiert sind und die aufgrund der lokalen Aminosäuresequenz flexibel genug sind, die Insertion einer Peptidsequenz zu überstehen. Dabei wurde sorgfältig darauf geachtet, daß keine sekundären Strukturelemente des AAV2-Kapsidproteins ausgewählt wurden, die das Kapsid destabilisieren würden.

Eine weitere Möglichkeit zur Bestimmung der Oberflächen-lokalisierten Bereiche der Strukturproteine ist ein Vergleich der für die Kapside kodierenden Nukleinsäuresequenzen von unterschiedlichen AAV-Serotypen. Hierzu können beispielsweise bekannte DNA-Sequenzen unterschiedlicher AAV-Serotypen, wie AAV2, AAV3, AAV4 oder AAV6, für Strukturanalysen möglicher Kapsidmorphologien von beispielsweise AAV2 herangezogen werden, wobei ab initio mögliche Tertiärstrukturen berechnet und Sequenzbereiche aufgrund allgemein bekannter Aminosäure-Eigenschaften den inneren oder äußeren Kapsidbereichen zugeordnet werden können. So konnten beispielsweise gemäß der vorliegenden Erfindung sieben mögliche Insertionsstellen im VP3-Bereich des AAV2-Kapsids ermittelt werden, die die Insertion beispielsweise eines Liganden und seine Expression auf der Virusoberfläche ermöglichten (siehe unten).

Beschrieben sind auch Mutation(en), die am N-Terminus des Strukturproteins lokalisiert sind, da gefunden wurde, daß beispielsweise bei den Parvoviren CPV und B19 der N-Terminus an der Zelloberfläche liegt. Dabei wird die Mutation vorzugsweise nicht direkt am N-Terminus von VP1 sondern einige Aminosäuren downstream vom N-Terminus vorgenommen.

In einer weiteren bevorzugten Ausführungsform verursacht die Mutation eine Änderung der Protein-Zellmembranrezeptor-Interaktion, wobei der Zellmembranrezeptor vorzugsweise ein Glykoprotein von ca. 150 kD und/oder ein Heparansulfat-Proteoglycan, wie oben bereits näher beschrieben, ist. Diese beiden Rezeptoren sind vermutlich Primärrezeptoren, die durch mindestens einen Sekundärrezeptor (s.o.) ergänzt werden.

Im allgemeinen kann die Mutation im VP1-, VP2- und/oder VP3-Strukturprotein liegen, wobei das VP1- und/oder das VP3-Strukturprotein bevorzugt sind. Des weiteren ist das mutierte Strukturprotein vorzugsweise weiterhin zur Partikelbildung, d.h. zur Ausbildung eines ikosaedrischen Kapsids, befähigt. Des weiteren kann das Strukturprotein von allen AAV-Serotypen abgeleitet sein, insbesondere von humanen Serotypen, vorzugsweise von AAV1, AAV2, AAV3, AAV4, AAV5 und/oder AAV6, vor allem von AAV2, AAV3 und/oder AAV6. Darunter fallen auch von den genannten Serotypen, insbesondere AAV2, abgeleitete Serotypen.

In einer weiteren bevorzugten Ausführungsform stellt/stellen die Mutation/en Punktmutation(en), die Mutation(en) mehrerer Aminosäuren, eine oder mehrere Deletionen und/oder eine oder mehrere Insertionen sowie Kombinationen dieser Mutationen im Strukturprotein dar, wobei die Insertion vorzugsweise die Insertion eines Zellmembranrezeptor-Liganden, ein Rep-Protein oder -Peptid, beispielsweise in Form einer Rep-Domäne, ein immunsuppressives Protein oder Peptid und/oder ein Protein oder Peptid mit einem Signal zur Doppelstrangsynthese des Transgens bzw. Fremdgens ist.

Beispiele von Insertionen sind u.a. Integrine, Cytokine oder Rezeptor-Bindungsdomänen von Cytokinen oder Wachstumsfaktoren, wie z.B. GM-CSF, IL-2, IL-12, CD40L, TNF, NGF, PDGF oder EGF, an Zelloberflächenrezeptoren bindende einzelkettige Antikörper, sog. "single chain" Antikörper (scFv), beispielsweise an die Oberflächenrezeptoren CD40, CD40L, B7, CD28 oder CD34 bindende einzelkettige Antikörper, oder Epitope bzw. Rezeptorbindungsstellen, die beispielsweise ihrerseits von bestimmten Antikörpern, beispielsweise Anti-CD40L-monoklonale Antikörper, bzw. von chemischen Substanzen oder Hormonen, z.B. Katecholamine, erkannt werden. Weitere Beispiele sind auch Antikörper gegen bestimmte Epitope wie beispielsweise "cell recognition particles" oder Teile von Xenobiotika, wie Drogen, die teilweise auf der Zelloberfläche bestimmter Zellen präsentiert werden.

In einer bevorzugten Ausführungsform werden antikörperbindende Strukturen, wie z.B. Protein A, Protein G oder anti-Fc-Antikörper, bzw. Teile hiervon, insertiert. An diese werden wiederum spezifische Antikörper gegen bestimmte Zelloberflächenstrukturen (beispielsweise gegen das CD40 bei lymphatischen Zellen oder gegen das CD34 bei hämatopoietische Zellen) angekoppelt. Damit wird ein fast universeller Einsatz von erfindungsgemäßes Strukturprotein enthaltenden Substanzen möglich, da praktisch jeder Antikörper ankoppelbar wäre, wobei der Einsatz dann auch sehr spezifisch erfolgen kann.

Bei diesem indirekten Targeting kann man einen universellen AAV-Targetingvektor herstellen, der individuell und je nach Anwendung mit unterschiedlichen Antikörpern beladen werden kann, die jeweils gegen verschiedene spezifische Oberflächenrezeptoren oder Oberflächenmoleküle der Zielzelle gerichtet sind und über die das Virus an die Zielzelle bindet und diese infizieren soll. Damit entfällt die individuelle Klonierung verschiedener AAV-Mutanten für gezielte Targetingprobleme. Dabei können entsprechende erfindungsgemäße Vektoren oder Kapsid-Mutanten über den Einsatz verschiedenster Antikörper auch dazu dienen, geeignete Oberflächenrezeptoren auf den Zielzellen zu bestimmen, die zur Virenbindung oder zu deren Aufnahme in Zellen geeignet sind, so daß schnell und effektiv Targetingrezeptoren auf den Zielzellen gescreent werden können.

Besonders bevorzugt ist es, ein- oder mehrfach - vorzugsweise einfach - die Z-Domäne von Protein A, insbesondere in verkürzter, deletierter Form, z.B. als Z34C Protein (Starovasnik et al. (1997), Proc.Natl.Acad.Sci. USA 16:94, 10080-10085), zu insertieren und dabei u.U. zuvor einige Aminosäuren an der Deletionsstelle im Kapsidprotein zu deletieren. Die Z-Domäne von Protein A und deren zweifache, sukzessive Insertion in das Kapsid von Sindbis-Viren ist bei Ohno et al. (1997) Nat. Biotech. 15, 763-767 beschrieben. Protein A bindet über fünf unabhängige Domänen an den FC-Teil von Antikörpern. Die am stärksten bindende Domäne ist die B- oder Z-Domäne, von der 33 Aminosäuren für die Bindung essentiell sind. Diese Bindungsstruktur kann durch zwei Cystein-Brücken stabilisiert werden (Starovasnik et al. supra).

Ein besonders bevorzugter Ligand ist beispielsweise das P1-Peptid (QAGTFALRGDNPQG), das ein 14 Aminosäuren langes Peptid aus der Core-Sequenz einer Alpha-Kette der Laminin-Familie darstellt. Diese Sequenz ist beispielsweise ausreichend, um einen Integrin-Rezeptor zu erkennen, der u.a. die Endozytose viraler Partikel, z.B. von Adenovirus, vermittelt. Das P1-Peptid bindet unabhängig von seiner Konformation (linear oder zirkulär) an den Integrin-Rezeptor. Gemäß der vorliegenden Erfindung wird die kodierende DNA-Sequenz des P1-Peptids in das Gen kodierend für ein Strukturprotein von AAV, welches beispielsweise auf einem Helferplasmid liegt, eingebaut. Nach der Verpackung mit dem mutanten Helferplasmid erhält man rekombinantes AAV mit P1 im Kapsid (rAAV-P1).

Weitere mögliche Liganden, die an den Insertionsstellen eingefügt werden sind solche, die lediglich über ihre Ladung, die Art der chakteristischen Aminosäurezusammensetzung, und/oder über ihre spezifische Glykosilierung und/oder Phosphorylierung an Zelloberflächenmoleküle binden. Dabei versteht man unter der Art der chakteristischen Aminosäurezusammensetzung, daß diese z.B. überwiegend hydrophobe, hydrophile, sterisch sperrige, geladene oder Amino-, Carbonsäure-, SH- oder OH-Gruppen enthaltende Aminosäurereste aufweisen. Damit können Zellen über einen unspezifischen Mechanismus für die AAV-Transfektion zugänglich gemacht werden. Dabei sind beispielsweise viele Zelloberflächenmoleküle spezifisch glykosiliert und phosphoryliert bzw. negativ geladen und können so z.B. ein Target für eine AAV-Mutante mit mehrfach positiv geladenem Aminosäureliganden darstellen.

Beschrieben sind auch Mutation(en) durch Insertionen an der XhoI-Schnittstelle der VP1-kodierenden Nukleinsäure oder an der BsrBI-Schnittstelle der VP1-kodierenden Nukleinsäure oder durch eine Deletion zwischen den BsrBI-HindII-Schnittstellen der VP1-kodierenden Nukleinsäure und eine oder mehrere Insertionen, vorzugsweise an der Stelle der Deletion.

Weiterhin beschrieben sind Mutation(en) durch eine oder mehrere Deletionen zwischen den XhoI-XhoI-Schnittstellen der VP1-kodierenden Nukleinsäure, die 62 Aminosäuren umfaßt (Hermonat, P. L. et al. (1984), J. Virol., 51, 329-339) oder Deletion(en) zwischen den BsrBI-HindII-Schnittstellen der VP1-kodierenden Nukleinsäure, die innerhalb der oben beschriebenen Deletion liegt und 29 Aminosäuren umfaßt. Diese Deletion hat den Vorteil, daß sie keine Überlappung mit dem rep-Gen hat und daher den Verpackungsmechanismus im wesentlichen nicht beeinflußt.

Erfindunggemäß liegen ein oder mehrere Insertionen im VP3-Strukturprotein (Rutledge, E. A. et al. (1998) supra) vor und/oder nach mindestens einer Aminosäure in der Sequenz ausgewählt aus YKQIS SQSGA, YLTLN NGSQA, YYLSR TNTPS, EEKFF PQSGV, NPVAT EQYGS, LQRGN RQAAT, NVDFT VDTNG, da diese Stellen an den exponierten Stellen eines Loops liegen, wobei das Risiko gering ist, die VP3-Struktur zu ändern.

Die Punktmutation(en), die Mutation(en) mehrerer Aminosäuren, die Deletion(en) oder Insertion(en) wird/werden nach allgemein bekannten Methoden durch Deletion und Insertion in dem für das Strukturprotein codierenden Gen durchgeführt. Die Deletionen lassen sich beispielsweise mittels PCR-unterstützter Mutagenese in die einzelnen Strukturprotein-Gene einführen. Die Insertionen lassen sich nach allgemein bekannten Methoden beispielsweise mittels Hydrolyse durch Restriktionsendonukleasen der entsprechenden Strukturprotein-Gene und anschließender Ligasereaktion einfügen.

Mittels geeigneter Zellen, die einen ausgewählten Rezeptor exprimieren, beispielsweise den Laminin-Alpha-Rezeptor, aber schlecht mit Wildtyp-AAV, beispielsweise mit Wildtyp-AAV2, zu infizieren sind, kann man in einem Adhäsionstest (Valsesia-Wittmann, S. et al. (1994) J. Virol. 68, 4609-4619) relativ einfach die Änderung der Infektiosität der erfindungsgemäBen, mutierten Strukturproteine, beispielsweise die funktionelle Expression des P1-Peptids auf der Oberfläche von AAV nachweisen. Der Vorteil dieses Testsystems ist, daß man schnell mittels Inspektion und quantitativ mittels Messung der optischen Dichte beispielsweise die Expression des P1-Peptids auf der viralen Oberfläche bestimmen kann.

Zum schnellen Screening der Expression inserierter Liganden auf der Virusoberfläche und von Modifikationen des Tropismus wurde daher auf der Grundlage des Laminin/Integrin-Ligand/Rezeptor-Systems ein geeignetes Targetingmodell entwickelt. Hierzu wurde die Nukleinsäure codierend für das bereits oben näher beschriebene P1-Peptid, welches unabhängig von seiner Konformation (linear oder zirkulär) an den Integrin-Rezeptor bindet, in das cap-Gen eingebaut, so daß man nach der Virusverpackung mit mutiertem AAV2-Genom rAAV mit P1-Liganden im Kapsid (rAAV-P1) erhielt. Zur Durchführung des Testsystems wurden zwei verschiedene Zellinien verwendet, die zum einen durch Wildtyp-AAV2 infizierbar sind und den AAV2-Rezeptor (Heparansulfat-Proteoglycan-Rezeptor, HPR, evtl. auch Sekundärrezeptoren (s.o.)), aber nicht den Integrin-Rezeptor für Laminin-P1 (LP1-R) exprimieren und zum anderen, die den LP1-R auf ihrer Oberfläche exprimieren, aber nicht HPR. Geeignete Zellinien lassen sich in Durchfluß-zytometrischen Untersuchungen mit Hilfe von Anti-HPR-Antikörpern und Adhäsions-Assays an Laminin-P1 ermitteln.

Diese Tests zeigten, daß beispielsweise die oben beschriebenen Mutanten die Laminin-Alpha-Rezeptor-positiven Indikatorzellen, z.B. die Zellinie M07-LP1-R mit mindestens 10-fach höherer Effizienz infizieren als Wildtyp-AAV. In Kompetitions-Assays mit löslichem P1-Peptid wurde beispielsweise auch gezeigt, daß die Infektion mit rAAV-P1 tatsächlich durch den inserierten Liganden vermittelt wurde. Ebenso wurde in einem weiteren Test mit einer rAAV-P1-Mutante B16F10-Zellen, eine Zellinie, die normalerweise nicht durch Wildtyp AAV infiziert wird, um mehr als vier Größenordnungen stärker transfiziert als dies mit dem Wildtyp AAV möglich war.

Ein anderer Gegenstand der vorliegenden Erfindung ist auch ein erfindungsgemäßes Strukturprotein in Form eines AAV-Partikels, insbesondere in Form eines AAV-Kapsids, da Partikel bzw. Kapside als Träger von ausgewählten Verbindungen, z.B. rAAV-Transduktionsvektoren, besonders geeignet sind.

Weitere Gegenstände der vorliegenden Erfindung sind eine Nukleinsäure, vorzugsweise eine RNA oder DNA, insbesondere eine doppelsträngige DNA, kodierend für ein erfindungsgemäßes Strukturprotein.

Die vorliegende Erfindung bezieht sich auch auf eine Zelle, vorzugsweise eine Säugetierzelle, beispielsweise eine COS-Zelle, Hela-Zelle oder 293-Zelle, enthaltend eine erfindungsgemäße Nukleinsäure. Derartige Zellen eignen sich beispielsweise zur Herstellung der rekombinanten AAV-Partikel.

Ein weiterer Gegenstand der vorliegenden Erfindung ist daher auch ein Verfahren zur Herstellung eines erfmdungsgemäßen Strukturproteins, insbesondere zur Herstellung eines erfindungsgemäßen Strukturproteins in Form eines AAV-Partikels, wobei eine geeignete Zelle, enthaltend eine Nukleinsäure, kodierend für das erfindungsgemäße Strukturprotein kultiviert und ggf. das exprimierte Strukturprotein isoliert wird. Beispielsweise läßt sich das erfindungsgemäße Strukturprotein über einen Cäsiumchlorid-Gradienten, wie beispielsweise in Chiorini, J. A. et al. (1995), supra, beschrieben, isolieren.

Ein weiterer Gegenstand der vorliegenden Erfindung bezieht sich auf die in-vitro- Verwendung des Erfindungsgemäßen Strukturproteins für die Änderung des Tropismus von AAV, für die Transformation einer Zelle, insbesondere einer Zelle, die vorher einer AAV-Infektion wenig zugänglich war, wie beispielsweise einer hämatopoetischen Zelle, für die Gentherapie in Form von geeigneten rAAV-Vektoren, wie oben bereits näher beschrieben, oder für das genomische Targeting.

Ebenfalls umfaßt ist die in-vitro- Verwendung eines erfindungsgemäßen Strukturproteins, bei dem die Mutation eine erhöhte Infektiosität gegenüber bestimmten Zellen, beispielsweise über einen Integrin-Rezeptor verfügende B16F10, bewirkt hat, für Wirksamkeitsuntersuchungen mit diesen Zellen. Beispiele sind Tumormodelle und - zelllinien vorzugsweise aus Maus. Bei dieser Verwendung können für den Menschen realistische und vergleichbare Modelle zur Untersuchung herangezogen werden, die so bisher nicht zugänglich waren wie bestimmte Mauszellinien. Dabei ist festzustellen, daß sich Mauszellen generell um ein Vielfaches schlechter infizieren lassen als humane Zellen. So sind in der Maus mit B16F10-Melanomzellen induzierte Tumore für AAV2 mit unmutiertem Capsid nicht zugänglich. Gerade in diesem Fall erlauben aber die erfindungsgemäßen Proteine AAV2-Wirksamkeitsstudien in diesem und entsprechend anderen Tumormodellen in Mäusen. Dabei tritt erleichternd hinzu, daß Mauszellen in vielen Geweben und Zelltypen über den spezifischen Integrinrezeptor für das P1-Peptid verfügen, das ein bevorzugter Ligand der erfindungsgemäß mutierten Strukturproteine ist. Mit den erfindungsgemäßen Mutanten kann somit durch die erhöhte Infektiosität für beispielsweise B16F10 und andere Tumorzellinien aus der Maus, die z.B. über diesen spezifischen Integrinrezeptor verfügen, ein für den Menschen realistischeres und vergleichbareres Test-Modell konstruiert werden als bisher bekannt, da die induzierten Tumore so wesentlich effizienter transduziert werden können.

Eine weitere in-vitro- Verwendung des erfindungsgemäßen Strukturproteins ist die Diagnostik. So können beispielsweise erfindungsgemäß Antikörper oder antikörperbindende Substanzen mit Antikörpern als Liganden eingesetzt werden, die bestimmte präsentierte Epitope auf Zellen, beispielsweise einer Blutprobe erkennen und binden, so daß ein Signal ausgelöst wird. Anwendungsbeispiele wären präsentierte Teile von Xenobiotika wie Drogen oder "cell recognition particles", mit denen die Herkunft von Gewebszellen, beispielsweise in der Tumordiagnose, bestimmt werden kann.

Andere Gegenstände der vorliegenden Erfindung beziehen sich auch auf ein Arzneimittel, bzw. Diagnostikum enthaltend ein erfindungsgemäßes Strukturprotein, eine erfindungsgemäße Nukleinsäure oder eine erfindungsgemäße Zelle und ggf. geeignete Hilfs- und Zusatzstoffe, wie z.B. eine physiologische Kochsalzlösung, Stabilisatoren, Proteinaseinhibitoren, etc.

Ein wesentlicher Vorteil der vorliegenden Erfindung ist, daß durch die erfindungsgemäße Mutagenese von Strukturproteinen von AAV die Infektiosität im wesentlichen ohne Verlust der Verpackungseffizienz rekombinanter AAV-Vektoren in das Kapsid des Virus geändert, insbesondere die Infektiosität für wenig zugängliche Zellen, wie z.B. hämatopoetische Zellen, um das Mehrfache erhöht werden kann. Die vorliegende Erfindung eignet sich daher im besonderen Maße für eine verbesserte in vitro wie auch in vivo Transformation bestimmter Zellen, beispielsweise für die somatische Gentherapie.

Die folgenden Beispiele und Abbildungen sollen die Erfindung näher erläutern, ohne sie zu beschränken.
Fig. 1) zeigt die Detektion von P1 auf der Oberfläche der Kapsid-Mutanten und des Wildtyps entweder im direkten ELISA (schwarze Balken) oder im indirekten ELISA (graue Balken).
Fig. 2) zeigt die Bindung der Kapsidmutanten bzw. des Wildtyps an unterschiedliche Zelltypen.
Fig 3) zeigt die Inhibition der Bindung der Kapsidmutante I-447 an B16F10-Zellen.
Fig 4) zeigt die Inhibition der Bindung der Kapsidmutante I-587 an B16F10-Zellen.

### Beispiele

Mittels PCR-unterstützter Mutagenese und Schneiden mit den Restriktionsenzymen XhoI, BsrBI bzw. HindIII wurden folgende Mutationen hergestellt:
1. Mutationen im VP1
   a) Deletion zwischen den XhoI-XhoI-Schnittstellen des VP-1 (ΔXho; 62 Aminosäuren, AS) (Hermonat et al. (1984) Journal of Virology 51, 329 - 339),
   b) Deletion zwischen BsrBI und HindII-Schnittstellen des VP-1, die innerhalb von der obigen Deletion a) liegt und 29 AS umfaßt (ΔBH);
   c) Deletion zwischen BsrBI und HindII, sowie Insertion eines Liganden (P1-Peptid) (ΔBH+L); und
   d) Reine Insertion des Liganden (P1-Peptid) an der BsrBI-Schnittstelle (B+L).
2. Mutationen im VP3
   a) ins447; YYLSR TNTPS (CPV: 300)
   b) ins534; EEKFF PQSGV (CPV: 390)
   c) ins573; NPVAT EQYGS (CPV: 426)
   d) ins587; LQRGN RQAAT (CPV: 440)
   e) ins713; NVDFT VDTNG (CPV: 565) CPV bedeutet hier die Lage im äquivalenten CPV-Kapsid
   (Nomenklatur nach Zahl der Aminosäuren (AS) gezählt nach den AS ab Beginn des N-Terminus im VP-1 von AAV2, umrahmt von jeweils 5 N-terminal davon gelegenen und 5 C-terminal davon gelegenen Aminosäuren; die AS, nach der die Insertion eingebracht wurde, ist in Fettschrift dargestellt).
   Es ist auch möglich, daß in die fünf unmittelbar benachbarten AS, die neben der fett markierten AS liegen, ebenfalls eine Insertion eingebracht wird, da diese ebenfalls innerhalb eines Loops im AAV2-Kapsid liegen.
3. Charakterisierung der Kapsidmutanten
   Nach Durchführung der Mutationen im AAV2-Genom und Verpackung der mutierten Viren mit LacZ-Reportergen wurden die physikalischen Vektor-Titer durch Dot-Blot und Kapsid-Titer mit A20-Antikörper-ELISA bestimmt und erste Infektionstests auf HeLa-Zellen durchgeführt. Dadurch konnte bestimmt werden, ob die Mutationen die Struktur der VP-Proteine oder die Interaktion zwischen verschiedenen VP-Proteinen so stören, daß eine Verpackung unterbleibt bzw. die Infektion der Zielzelle gestört wird (Tabelle 1).

**Tabelle 1 Verpackungseffizienz der hergestellten Virusmutanten**

| Virusstock | physikalische Virustiter | Kapsid-Titer (ELISA mit A20-MAb) |
|---|---|---|
| Wildtyp-Kapsid | 1.10¹² | 1.10¹¹ |

| VP1-Mutanten | | |
|---|---|---|
| ΔXho | 6.10¹² | 5.10¹⁰ |
| ΔBH | 8.10¹¹ | 4.10⁹ |
| ΔBH+L | 1.10¹³ | 5.10¹⁰ |
| B+L | 3.10¹² | 5.10⁹ |

| VP3-Mutanten | | |
|---|---|---|
| 300 **(I-447)** | 1.10¹² | 4.10¹⁰ |
| 390 **(1-534)** | 1.10¹⁰ | 1.10⁷ |
| 426 **(I-573)** | 3.10¹⁰ | 1.10⁷ |
| 440 **(I-587)** | 1.10¹² | 2.10¹⁰ |
| 565 **(I-713)** | 5.10¹⁰ | 1.10⁷ |

Gezeigt sind die physikalischen Virustiter (Dot-Blot) und Kapsid-Titer (A20-Kapsid-EUSA). Die Konzentrationen sind in Partikel/ml angegeben.

Ergebnis:
Für alle 4 VP1-Mutanten, die rekombinante Vektoren mit LacZ-Transgen sind, konnte gezeigt werden, daß Mutationen die Verpackungseffizienz nicht beeinflussen und alle mutierten Viren mit ähnlichen guten Titern wie Vektoren mit unmutiertem Kapsid verpackt werden können (⁻ 10¹² Partikel/ml). Auch die AAV-Vektoren mit Mutationen im VP3-Bereich konnten mit LacZ-Reportergen erfolgreich verpackt werden (10¹⁰-10¹² physikalische Partikel/ml).
4. Bindung von rAAV-P1 an Laminin-Rezeptor-positive Indikator-Zellen
   Die oben näher beschriebenen Adhäsionstests zeigten, daß die obigen Mutanten in mindestens einem Fall die Laminin-alpha-Rezeptor-positiven Indikatorzellen, z.B. die Zellinie M07-LP1-R mit mindestens 10fach höherer Effizienz infizieren als Wildtyp-AAV. In Kompetitions-Assays mit löslichem P1-Peptid wurde zudem gefunden, daß die Infektion mit rAAV-P1 tatsächlich durch den inserierten Ligand vermittelt wird.
5. P1-Mutation im VP3
   Zunächst wurde von einem Plasmid pUC-AV2, das durch Subklonierung des 4.8-kb BglII-Fragments des pAV2 (ATCC 37261, ref. 53) in die BamHI Schnittstelle des pUC19 (New England BioLabs Inc.) hergestellt wurde, ausgegangen. Mittels dem Fachmann bekannter PCR-unterstützter Mutagenese wurden an definierten Stellen des Plasmids Mutationen vorgenommen. Dabei wurde eine für P1, ein 14-AS-Peptid, mit der AS-Sequenz QAGTFALRGDNPQG, das das RGD-Bindungsmotiv eines Lamininfragments (Aumailly et al. (1990) FEBS Lett. 262, 82-86) enthält, codierende Sequenz nach den Nukleotiden 3543, 3804, 3921 und 3963 eingefügt. Dies entspricht einer Insertion nach den Aminosäuren 447, 534, 573 und 587 des AAV2-Kapsidproteins (Nomenklatur nach Zahl der Aminosäuren (AS) gezählt nach den AS ab Beginn des N-Terminus im VP-1 von AAV2). In der anschließenden PCR werden jeweils 2 mutationsspezifische Primer und als Matrize ein Plasmid, pCap verwendet, das nur das cap-Gen enthält und dadurch gebildet wird, daß das 2.2 kb EcoRI-BspMI-Fragment aus pUC-Av2 herausgeschnitten und in die EcoRI-Schnittstelle des pUC19 eingefügt wird. Anschließend werden die PCR Produkte in Bakterien amplifiziert, sequenziert und das 1.4-kb EcoNI-XcmI-Fragment, das P1 enthält in pUC-AV2, in dem die korrespondierende Wildtyp-cap-Sequenz herausgeschnitten wurde, subkloniert. Dementsprechend enthielten die nach den AS-Insertionsstellen pI-447, pI-534, pI-573 und pI-587 genannten Plasmide (Mutanten) das komplette AAV2-Genom.
6. Herstellung von AAV2-Partikel
   HeLa-Zellen (eine humane Cervix-Epithel-Zellinie) wurden mit den Plasmiden transfiziert, dann ca. 20h inkubiert und anschließend mit Adenovirus Typ 5 infiziert. 72 h nach der Infektion wurden die Zellen aufgeschlossen und AAV2-Partikel über einen CsCl-Gradienten gereinigt.
7. Charakterisierung der Kapsidmutanten gemäß Beispiel 5
   In diesen Versuchen sollte festgestellt werden, ob die Kapsidmutanten das virale Genom verpacken und vollständige Kapside zu bilden können. AAV2-Partikel der Mutanten gemäß Beispiel 5 wurden zunächst darauf überprüft, ob und wenn ja wieviele Partikel das Virus-Genom tragen und wieviel DNA in den Kapsid-Mutanten verpackt war. Dazu wurden die gemäß Beispiel 6 gereinigten Viren (Mutanten und Wildtyp) mit DNAse behandelt, geblottet und mit einer Rep-Sonde hybridisiert. Die in Tabelle 2 dargestellten Titer sind Titer von AAV2 Partikeln mit mutiertem Kapsid und Wildtyp-Gen, das die entsprechende Liganden-Insertion trägt, im Gegensatz zu Tabelle 1, die den Titer von AAV2-Mutanten mit LacZ-Reportergen (Transgen) zeigt.

Der sich daraus ergebende Titer zeigte keine qualitative Differenz im Veigleich zum Wildtyp, auch wenn quantitative Unterschiede zu sehen sind, die aber wiederum so gering sind, das keine für die Verpackung essentiellen Domänen durch die Mutationen funktionell ausgeschaltet sein können (s. Tabelle 2).

Aus diesem Ergebnissen konnten noch keine Information über die Konformation des Kapsids abgelesen werden. In einem weiteren Experiment wurden A20 monoklonale Antikörper (A20MAb) in einem ELISA eingesetzt. A20MAb reagieren spezifisch nur mit komplett zusammengesetztem AAV2-Kapsid, nicht mit freiem Kapsid-Protein (Wistuba et al., (1997), J. Virol. 71, 1341-1352). Auch hier sind die Ergebnisse in Tabelle 2 dargestellt. Wieder zeigt der sich daraus ergebende Titer keine entscheidende quantitative oder qualitative Differenz im Vergleich zum Wildtyp. Das zeigt, daß die Insertionen an strukturell irrelevanten Loops erfolgt war, ohne daß dort die Insertion von P1 eine Veränderung ausgelöst hatte. Im Hinblick auf ihre Fähigkeit, DNAenthaltende Partikel zu bilden, konnten die Mutationen in zwei Gruppen eingeteilt werden (Tabelle 2): In der einen Gruppe (Mutanten 1-447 und I-587) war die Fähigkeit zur Ausbildung DNA-enthaltender Partikel dem Wildtyp AVV2 entsprechend. In der zweiten Gruppe war diese Fähigkeit um zwei Größenordnungen kleiner (Mutanten 1-534 und 1-573). Diese Ergebnisse konnten durch Elektronenmikroskopanalyse bestätigt werden.

**Tabelle 2 Verpackungseffizienz der hergestellten Virusmutanten gemäß Beispiel 5**

| Virusstock | physikalische Virustiter | Kapsid-Titer (ELISA mit A20-MAb) |
|---|---|---|
| Wildtyp-Kapsid | 8.10¹³ | 6.10¹² |

| Mutanten | | |
|---|---|---|
| 1-447 | 1.10¹³ | 8.10¹¹ |
| I-534 | 5.10¹¹ | 3.10¹⁰ |
| I-573 | 1.10¹² | 1.10¹¹ |
| I-587 | 4.10¹³ | 3.10¹² |

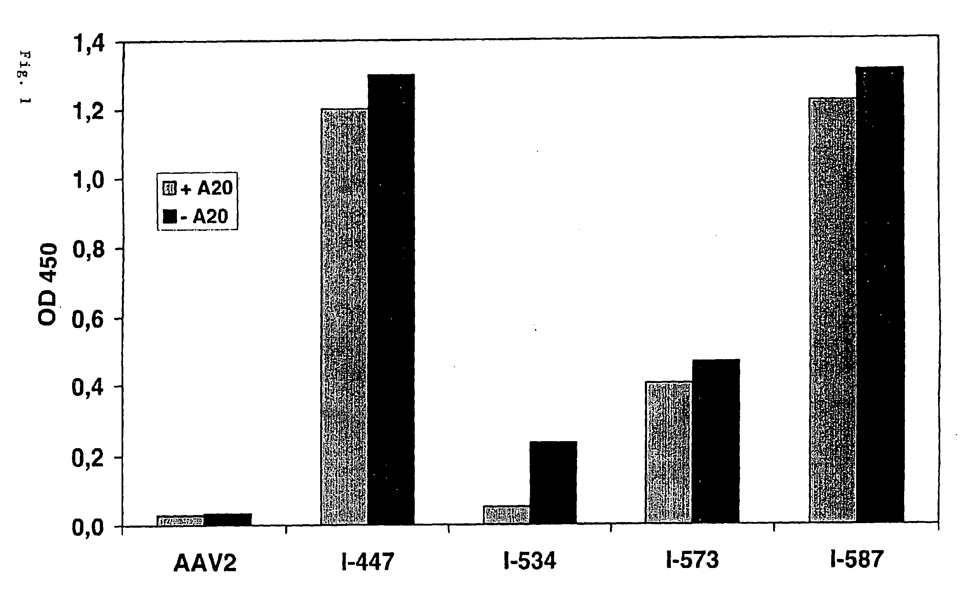

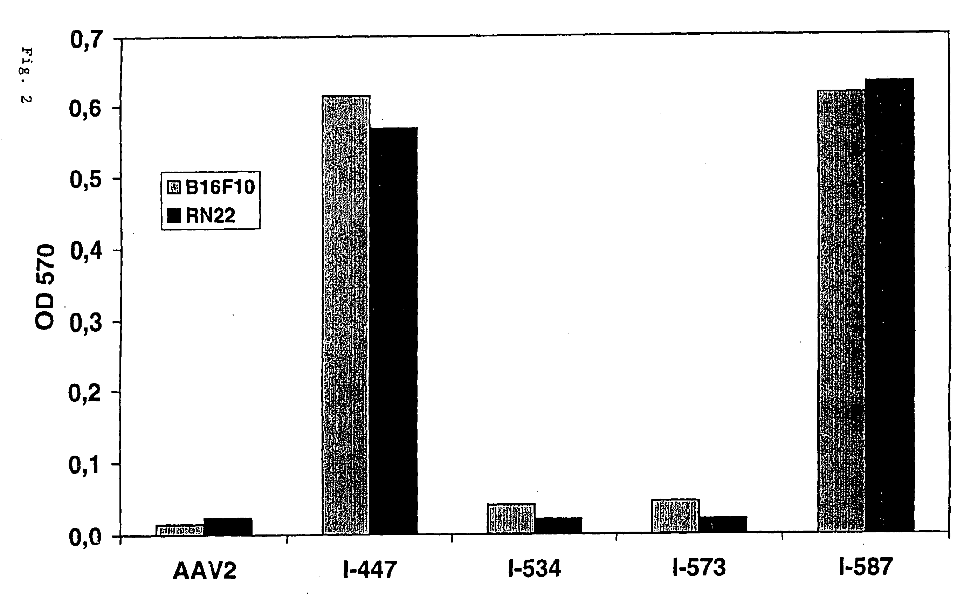

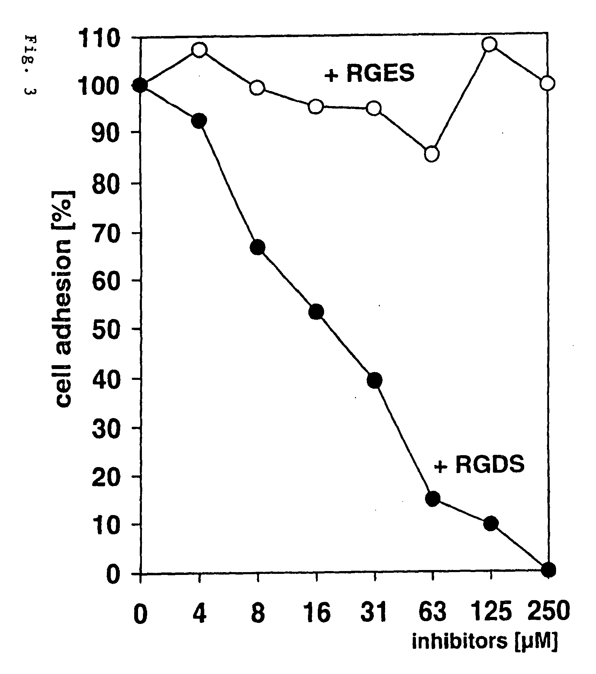

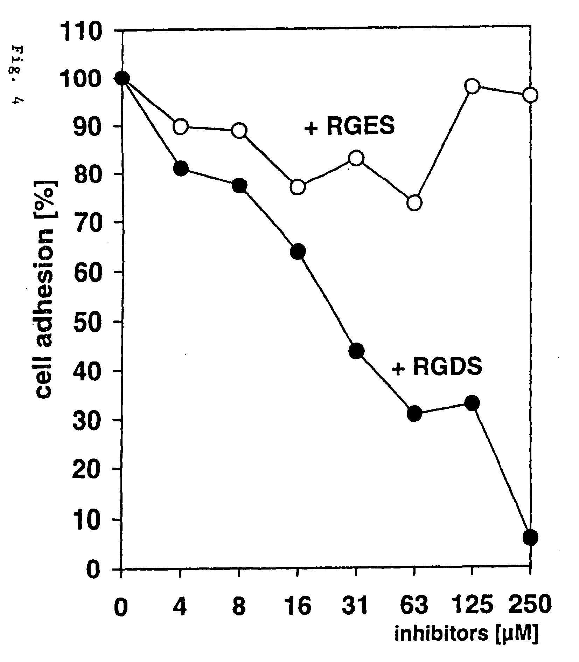

Gezeigt sind die physikalischen Virustiter (Dot-Blot) und Kapsid-Titer (A20-Kapsid-ELISA). Die Konzentrationen sind in Partikel/ml angegeben.
8. Expression von P 1 auf der Kapsid-Oberfläche
   Anschließend wurde untersucht, ob P1 auf der Kapsid-Oberfläche exponiert ist. Dazu wurden zwei verschiedene ELISA mit anti-P1-polyklonalen Antikörpern durchgeführt. Bei einem "direkt" genannten ELISA wurden die ELISA-Platten direkt mit den Viruspartikel in PBS über Nacht beschichtet, geblockt und mit dem anti-P1-polyklonalen Antikörper inkubiert. Kontrollen waren PBS (negativ) und ein Laminin-Fragment (positiv). Im indirekten Test wurden die Platten zuerst mit A20MAb beschichtet, dann die Viruspartikel und anschließend der anti-P1-polyklonale Antikörper zugegeben. Beim direkten Test zeigten I-447 und I-587 eine sehr deutliche, I-534 und I-573 hingegen nur eine schwache Reaktion. Beim indirekten Test hingegen zeigten 1-447, 1-587 und I-573 eine sehr deutliche, I-534 hingegen gar keine Reaktion (s. Fig 1).
9. Bindung von AAV2-Kapsidmutanten an Integrin exprimierende Zellen
   Die Bindung der Mutanten an den Integrinrezeptor wurde durch einen Zellanhaftungstest bestimmt, wobei dieser für virale Präparationen adaptiert wurde (Aumailly et al., Supra; Valsesia-Wittmann (1994); J. Virol. 68, 4609-4619) 1 x 10⁹ virale Partikel wurden in 100 µl PBS direkt auf 96er Mikrotiterplatten aufbeschichtet und mit PBS, das 1 % BSA enthielt, blockiert. Kontrollen wurden mit einem Laminin-Fragment mit einer Konzentration von 40 µg/ml (positive Kontrolle) oder mit BSA (10 mg/ml; negative Kontrolle) beschichtet. 1 x 10⁵ Zellen pro 100 µl wurden in den beschichteten Wells zugegeben. Sie wurden 30 Minuten bei 37° in einem wasserbefeuchteten Inkubator inkubiert, um sich anzuheften. Am Ende der Anheftungszeit wurden die Wells zweimal mit PBS ausgewaschen, um nicht angehaftete Zellen zu entfernen. Anhaftende Zellen wurden mit 100% Ethanol für 10 Minuten fixiert, mit Kristallviolett angefärbt und durch einen ELISA-Reader bei 570 nm quantifiziert. Es wurden diesmal B16F10-Zellen und RN22-Zellinien gewählt, da diese P1-spezifisches Integrin auf ihrer Oberfläche exprimieren und resistent gegen AAV2-Infektionen sind (Maass, G. et al. (1998) Hum. Gen. Ther., 9, 1049-1059; Aumailly et al., supra). Zwei der Mutationen, I-447 und 1-587 banden mit ähnlich hoher Effektivität sowohl an B16F10- und RN22-Zellen. Im deutlichen Gegensatz dazu wurde keine Bindung des Wildtyps AAV2 und der Mutanten 1-534 und 1-573 an diese Zellen gefunden (Fig. 2).
   In einem Inhibitionstest wurden Zellen mit RGDS oder RGES, löslichen synthetischen Peptiden mit variierender Konzentration, (1-250 µmol) gemischt, bevor sie auf die Platte aufgetragen wurden. Dieser Versuch wurde unternommen, um die Spezifität der Bindung der Mutanten 1-447 und 1-587 an B16F10-Zellen zu testen. Daher wurden die Zelladhäsionstests in der Gegenwart eines um die Bindungsstelle konkurrierenden Peptids (RGDS), welches der aktiven P1-Stelle entspricht und in Gegenwart eines inaktiven RGES-Peptid durchgeführt. Die Bindung beider Mutationen 1-447 und 1-587 an B16F10-Zellen konnte mit 30 µmol des RGDS-Peptids mit einer Effektivität von 50 % erreicht werden, während das RGES-Peptid selbst bei höheren Konzentrationen inaktiv war. Bei einer Konzentration von 250 µmol unterband das RGDS-Peptid komplett die Virusbindung an B16F10-Zellen (Fig. 3 und 4). Ähnliche Ergebnisse wurden mit RN22-Zellen erreicht.
10. Infektionstests mit Mutanten gemäß Beispiel 5
   Um den Tropismus der Kapsidmutanten I-447 und 1-587 zu testen, wurden Zellinien, Co-115 und B16F10, mit den mutierten Viren infiziert. Co-115-Zellen wurden zum Testen des Wildtyprezeptor-Tropismus der Virionen verwendet, da diese Zellen mit Wildtyp AAV2 transduziert werden können und das P1-Peptid nicht binden. Die B16F10-Zellinie wurde aus den in Beispiel 9 bereits genannten Gründen verwendet. Drei Tage nach der Infektion wurden die Zellen durch Immunofluoreszenzmessung mit Hilfe eines anti-Rep-Antikörpers darauf untersucht, ob das virale Rep-Protein exprimiert wird (Wistuba et al. (1997) J. Virol. 71, 1341-1352; Wistuba et al. (1995) J. Virol. 69, 5311-5319). Zellen wurden auf Objektträgern zu 70 % Konfluenz gezüchtet und mit verschiedenen Konzentrationen erfindungsgemäßer viraler Präparationen in serumfreiem Medium zusammen mit Adenovirus 5 inkubiert. Die Titer der viralen Präparationen wurden drei Tage später entweder durch in-situ-Detektion der Rep-Proteinsynthese in einem Immunofluoreszenzassay (Rep-Titer) bestimmt. Dabei wurde die Immunofluoreszenzanfärbung mit AAV2-infizierten Zellen nach einer Methode von Wistuba et al. (Wistuba et al. (1997) J. Virol. 71, 1341-1352; Wistuba et al. (1995) J. Virol. 69, 5311-5319) durchgeführt. Die Objektträger wurden einmal mit PBS gewaschen, in Methanol (5 min, 4°C) fixiert und anschließend mit Aceton (5 min, 4°C) behandelt. Die Zellen wurden dann für eine Stunde bei Raumtemperatur mit dem monoklonalen Antikörper 76-3, der mit Rep-Proteinen von AAV2 reagiert, inkubiert. Anschließend wurde gewaschen und für eine Stunde mit einem Rhodamin-konjugierten Anti-Maus-sekundären Antikörper bei einer Verdünnung von 1:50 in PBS mit 1% BSA inkubiert. Die Titer wurden aus der letzten limitierenden Verdünnung der viralen Stammlösung errechnet, die zu fluoreszenzpositiven Zellen geführt hatten.
   Rep-positive CO115-Zellen konnten nach Infektion mit Wildtyp AAV2 und mit beiden Mutanten I-447 und 1-587 detektiert werden. Bei Co115-Zellen war die Infektiosität von 1-587 und I-447 um zwei bis drei Größenordnungen kleiner als die des Wildtyps (Tabelle 3). Die Transfektion von B16F10-Zellen war mit 1-447 genauso ineffektiv wie die mit Wildtypvirus (Tabelle 3). In klarem Gegensatz dazu sind nach Infektion mit 1-587 rep-positive B16F10-Zellen feststellbar, wobei der Titer des 1-587-Virus auf 1 x 10⁶ Rep-EFU/ml bestimmt wurde (Tabelle 3).
   Um zu untersuchen, ob die Transfektion von B16F10-Zellen durch die Mutante 1-587 spezifisch durch die Interaktion zwischen der P1-Sequenz auf der Oberfläche des mutierten Kapsids und dem Integrinrezeptor auf der Oberfläche der B16F10-Zellen vermittelt wurde, wurden die Zellen entweder mit dem konkurrierenden RGDS oder dem inaktiven RGES-Peptid bei Konzentrationen von 200 µmol vor der Infektion mit dem Virus inkubiert. Die Zugabe von RGDS-Peptid neutralisierte die Infektiosität von 1-587 auf B 16F 10-Zellen (Tabelle 3), während das Kontrollpeptid RGES keinen Effekt hatte.

**Tabelle 3: Virustiter auf der Zelloberfläche**

| Virusstock | Titer auf CO115-Zellen | Titer auf B16F10-Zellen | |
|---|---|---|---|
| | | - RGDS | + RGDS |
| Wildtyp-Kapsid | 2.10⁹ | <1 | nb |

| Mutanten | | | |
|---|---|---|---|
| I-447 | 1.10⁶ | <1 | nb |
| I-587 | 1.10⁷ | 1.10⁶ | <1 |
| rAAV/LacZ | 5.10⁷ | <1 | nb |
| rAAV(I-587)/LacZ | 6.10⁵ | 5.10⁴ | <1 |

Gezeigt sind die Titer auf den wildtypanfälligen CO115-Zellen und den wildtypresistenten B16F10-Zellen. Die Titer sind für 1-447 und 1-587 wie den Wildtyp in Rep-EFU/ml und für rAAV/LacZ und rAAV(I-587)/LacZ in LacZ-EFU/ml ausgedrückt. Dabei bedeutet EFU expressionsbilende Einheiten (Expressing Forming Unit) und nb heißt "nicht bestimmt".

In einem ergänzenden Versuch wurde, um auszuschliessen, daß die Infektion von B16F10 Zellen durch die Mutante 1-587 zusätzlich durch den Primärrezeptor Heparansulfat-Proteoglycan vermittelt wird, ein Kompetitionstest mit Heparin, einem Rezeptoranalogen, durchgeführt. Bei B16F10-Zellen konnte nach Gabe von Heparin keine Veränderung des infektiösen Titers, also der Infektiosität von 1-587, festgestellt werden. Im Gegensatz dazu konnte die Infektion von CO155-Zellen ab Heparinzugaben von 50 µg/ml Infektionsmedium vollständig blockiert werden. Daraus folgt, daß die Infektion unabhängig von Heparansulfat-Proteoglycan über P1-Liganden und den Integrin-Rezeptor erfolgt.
11. Infektionsassay der Mutanten gemäß Beispiel 5 mit Galaktosidase
   In einem weiteren an Beispiel 10 angelehnten Versuch wurden rAAV-Vektoren mit einem LacZ-Reportergen hergestellt, die entweder den Wildtyp (rAAV-Virion) oder 1-587 (rAAV(I-587)-Virion) enthielten. Die Viralpräparationen wurden rAAV/LacZ und rAAV(I-587)/LacZ genannt und zur Infektion von B16F10- bzw. CO115-Zellen (Kontrollen) verwendet.
   Infizierte Zellen wurden drei Tage nach der Infektion durch X-Gal-Anfärbung auf β-Galaktosidase-Expression getestet. Dabei wurde der X-Gal-in-situ-Test zur zytochemischen Anfärbung (LacZ-Titer) verwendet. Nach diesem wurden die Zellen, um die Expression von β-Galaktosidase zu testen, einmal in PBS gewaschen und dann mit 1,5 % Glutaraldehyd fixiert. Anschließend wurden die Zellen mit X-Gal (5-bromo-4-chloro-3-indolyl-β-D-Galaktopyranosid) behandelt, wie bereits von Chiorini et al. (1995) Hum. Gen. Ther. 6, 1531-1541, beschrieben. Die Titer wurden aus der letzten limitierenden Verdünnung der viralen Stammlösung errechnet, die zu β-Galaktosidase-produzierenden Zellen geführt hatte.
   Bei den Kontrollen an CO115-Zellen waren beide Virionen infektiös, allerdings rAAV (I-587)/LacZ um 2 Größenordnungen weniger effektiv. Bei Typ B16F10 wurden - wie erwartet - nach der Infektion mit rAAV/LacZ keine β-Galaktosidase-positiven Zellen gefunden. Nach der Infektion mit rAAV(I-587)/LacZ hingegen fanden sich überraschenderweise deutlich viele β-Galaktosidase-positive Zellen. Der Titer von rAAV-(I-587)/LacZ wurde mit 5 x 10⁴ LacZ-EFU pro ml bestimmt. Die Infektiosität von rAAV-Vektoren gegenüber B 16F 1 0-Zellen wurde durch die erfindungsgemäße Mutation um mehr als vier Größenordnungen verbessert (Tabelle 3).
   In einem ergänzenden Versuch wurde, um auszuschliessen, daß die Infektion von B16F10 Zellen durch die Mutante 1-587 zusätzlich durch den Primärrezeptor Heparansulfat-Proteoglycan vermittelt wird, ein Kompetitionstest mit Heparin, einem Rezeptoranalogen, durchgeführt. Bei B16F10-Zellen konnte nach Gabe von Heparin keine Veränderung des infektiösen Titers, also der Infektiosität von I-587, festgestellt werden. Im Gegensatz dazu konnte die Infektion von CO155-Zellen ab Heparinzugaben von 50 µg/ml Infektionsmedium vollständig blockiert werden. Daraus folgt, daß die Infektion unabhängig von Heparansulfat-Proteoglycan über P1-Liganden und den Integrin-Rezeptor erfolgt.
12. Z34C-Protein A-Mutation in VP3
   Es wurden analog zum Beispiel 5 an den dort erwähnten Stellen verschiedene Mutationen im VP3 vorgenommen, wobei eine für die Z34C-Domäne von Protein A (Starovasnik 1997 supra) kodierende Sequenz nach den Nukleotiden 3543, 3804, 3921 und 3963 eingefügt. Dabei wurden jeweils ein oder mehrere an der Insertionsstelle liegende Aminosäuren deletiert, um Probleme durch zu lange Insertionen zu vermeiden. Die Herstellung der Mutanten erfolgt wie bereits in Beispiel 5 ausgeführt. Anschließend wurden nach gleicher Vorgehensweise wie in Beispiel 6 entsprechende AAV2-Partikel hergestellt.

## Patentansprüche

1. Strukturprotein von Adeno-assoziiertem Virus (AAV), **dadurch gekennzeichnet, dass** das Strukturprotein mindestens eine Mutation enthält, die durch ein oder mehrere Insertionen bewirkt wird/werden, wobei jede der Insertionen in VP3 unmittelbar vor und/oder nach einer Aminosäure in der Sequenz ausgewählt aus YKQIS SQSGA, YLTLN NGSQA, YYLSR TNTPS, EEKFF PQSGV, NPVAT EQYGS, LQRGN RQAAT und NVDFT VDTNG lokalisiert ist.

2. Strukturprotein nach Anspruch 1, **dadurch gekennzeichnet, dass** die Mutation eine Erhöhung der Infektiosität des Virus bewirkt.

3. Strukturprotein nach Anspruch 1 oder 2, **dadurch gekennzeichnet, dass** die Mutation an der Virusoberfläche lokalisiert ist.

4. Strukturprotein nacheinem der Ansprüche 1 bis 3, **dadurch gekennzeichnet, dass** das mutierte Strukturprotein eine Änderung der Protein-Zellmembranrezeptor-Interaktion bewirkt.

5. Strukturprotein nach Anspruch 4, **dadurch gekennzeichnet, dass** der Zellmembranrezeptor ein Glykoprotein von ca. 150 kD und/oder ein Heparansulfat-Proteoglycan ist.

6. Strukturprotein nach einem der Ansprüche 1 bis 5, **dadurch gekennzeichnet, dass** das mutierte Strukturprotein zur Partikelbildung fähig ist.

7. Strukturprotein nach einem der Ansprüche 1 bis 6, **dadurch gekennzeichnet, dass** es von AAV2, AAV3, AAV4, AAV5 und/oder AAV6 stammt.

8. Strukturprotein nach einem der Ansprüche 1 bis 7, **dadurch gekennzeichnet, dass** die Insertion ein Zellmembranrezeptor-Ligand, ein Rep-Protein oder - Peptid, ein immunsuppressives Protein oder Peptid und/oder ein Protein oder Peptid mit einem Signal zur Doppelstrangsynthese des Fremdgens ist.

9. Strukturprotein nach Anspruch 8, **dadurch gekennzeichnet, dass** der Ligand ausgewählt ist aus einem Integrin, einem Cytokin oder einer Rezeptor-Bindungsdomäne von einem Cytokin, Integrin oder Wachstumsfaktor, an einem Zelloberflächenrezeptor bindenden einzelkettigen Antikörper, einem Antikörper gegen Zelloberflächenstrukturen, einer antikörperbindenden Struktur oder einem Epitop sowie aus Liganden, die über ihre Ladung, die Art der charakteristischen Aminosäurezusammensetzung und/oder über ihre spezifische Glykosilierung und/oder Phosphorylierung an Zelloberflächenmoleküle binden.

10. Strukturprotein nach einem der Ansprüche 1 bis 9, wobei das Strukturprotein ausgewählt ist aus mutiertem VP1, mutiertem VP2 oder mutiertem VP3.

11. Strukturprotein gemäß einem der Ansprüche 1 bis 10 in Form eines AAV-Partikels, insbesondere in Form eines AAV-Kapsids.

12. Nukleinsäure, kodierend für ein Strukturprotein gemäß einem der Ansprüche 1 bis 11.

13. Isolierte Zelle, mit Ausnahme einer menschlichen embryonalen Stammzelle, enthaltend eine Nukleinsäure gemäß Anspruch 12.

14. Verfahren zur Herstellung eines Strukturproteins gemäß einem der Ansprüche 1 bis 11, **dadurch gekennzeichnet, dass** eine Zelle gemäß Anspruch 13 kultiviert und ggf. das exprimierte Strukturprotein isoliert wird.

15. Arzneimittel, enthaltend ein Strukturprotein gemäß einem der Ansprüche 1 bis 11, eine Nukleinsäure gemäß Anspruch 12 und/oder eine Zelle gemäß Anspruch 13.

16. Diagnostikum, enthaltend ein Strukturprotein gemäß einem der Ansprüche 1 bis 11, eine Nukleinsäure gemäß Anspruch 12 und/oder eine Zelle gemäß Anspruch 13.

17. In-vitro-Verwendung eines Strukturproteins gemäß einem der Ansprüche 1 bis 11 für die Änderung des Tropismus von AAV, für die Transformation einer Zelle, für die Diagnostik, für Wirksamkeitsuntersuchungen und/oder für das genomische Targeting.

## Claims

1. A structural protein of the adeno-associated virus (AAV), **characterised in that** the structural protein contains at least one mutation effected by one or more insertions, wherein each insertion in VP3 is located immediately in front of and/or after an amino acid in the sequence selected from YKQIS SQSGA, YLTLN NGSQA, YYLSR TNTPS, EEKFF PQSGV, NPVAT EQYGS, LQRGN RQAAT, and NVDFT VDTNG.

2. The structural protein according to claim 1, **characterised in that** the mutation increases the infectiousness of the virus.

3. The structural protein according to claim 1 or 2, **characterised in that** the mutation is located on the virus surface.

4. The structural protein according to any one of claims 1 to 3, **characterised in that** the mutated structural protein effects a change in the protein/cell membrane receptor interaction.

5. The structural protein according to claim 4, **characterised in that** the cell membrane receptor is a glycoprotein of approx. 150 kD and/or heparan sulfate proteoglycan.

6. The structural protein according to any one of claims 1 to 5, **characterised in that** the mutated structural protein is capable of particle formation.

7. The structural protein according to any one of claims 1 to 6, **characterised in that** it originates from AAV2, AAV3, AAV4, AAV5 and/or AAV6.

8. The structural protein according to any one of claims 1 to 7, **characterised in that** the insertion is a cell membrane receptor ligand, a rep protein or peptide, an immunosuppressive protein or peptide, and/or a protein or peptide having a signal for double strand synthesis of the foreign gene.

9. The structural protein according to claim 8, **characterised in that** the ligand is selected from an integrin, a cytokine or a receptor-binding domain of a cytokine, integrin or growth factor, a single-chain antibody which binds to a cell surface receptor, an antibody against cell surface structures, an antibody-binding structure or an epitope as well as from ligands which bind to cell surface molecules due to their charge, the nature of their characteristic amino acid composition and/or due to their specific glycosylation and/or phosphorylation.

10. The structural protein according to any one of claims 1 to 9, **characterised in that** the structural protein is selected from mutated VP1, mutated VP2, and/or mutated VP3.

11. The structural protein according to any one of claims 1 to 10 in the form of an AAV particle, in particular in the form of an AAV capsid.

12. A nucleic acid encoding a structural protein according to any one of claims 1 to 11.

13. An isolated cell except for a human embryonic stem cell containing a nucleic acid according to claim 12.

14. A method for the production of a structural protein according to any one of claims 1 to 11, **characterised in that** a cell according to claim 13 is cultured and the expressed structural protein is optionally isolated.

15. A pharmaceutical preparation containing a structural protein according to any one of claims 1 to 11, a nucleic acid according to claim 12 and/or a cell according to claim 13.

16. A diagnostic preparation containing a structural protein according to any one of claims 1 to 11, a nucleic acid according to claim 12 and/or a cell according to claim 13.

17. In vitro use of a structural protein according to any one of claims 1 to 11 for modifying AAV tropism, for transforming a cell, for diagnostics, for efficacy testing and/or for genomic testing.

## Revendications

1. Protéine de structure de virus adéno-associé (AAV), **caractérisée en ce que** la protéine de structure comporte au moins une mutation, qui est provoquées par une ou plusieurs insertions, et **en ce que** chacune insertion est localisée dans VP3 immédiatement avant et / ou dernière un acide aminé dans la séquence sélectionnée parmi de YKQIS SQSGA, YLTLN NGSQA, YYLSR TNTPS, EEKFF PQSGV, NPVAT EQYGS, LQRGN RQAAT, et NVDFT VDTNG.

2. Protéine de structure selon la revendication 1, **caractérisée en ce que** la mutation provoque une augmentation de l'infectiosité du virus.

3. Protéine de structure selon la revendication 1 ou 2, **caractérisée en ce que** la mutation est localisée à la surface du virus.

4. Protéine de structure selon l'une des revendications 1 à 3, **caractérisée en ce que** la protéine de structure mutée provoque une modification de l'interaction récepteur de la membrane cellulaire - protéine.

5. Protéine de structure selon la revendication 4, **caractérisée en ce que** le récepteur de la membrane cellulaire est une glycoprotéine d'environ 150 kD et / ou un héparane-sulfate protéo-glycane.

6. Protéine de structure selon l'une des revendications 1 à 5, **caractérisée en ce que** la protéine de structure mutée est capable de former des particules.

7. Protéine de structure selon l'une des revendications 1 à 6, **caractérisée en ce qu'**elle provient de AAV2, AAV3, AAV4, AAV5 et / ou AAV6.

8. Protéine de structure selon l'une des revendications 1 à 7, **caractérisée en ce que** l'insertion est un ligand de récepteur de la membrane cellulaire, une protéine ou un peptide de réplication, une protéine immunosuppressive ou un peptide immunosuppressif, et / ou une protéine ou un peptide pourvu d'un signal déclenchant la synthèse d'ADN du gène étranger.

9. Protéine de structure selon la revendication 8, **caractérisée en ce que** le ligand est sélectionné parmi une intégrine, une cytokine, ou un domaine de liaison au récepteur provenant d'une intégrine, d'une cytokine, ou d'un facteur de croissance, d'un anticorps à chaîne unique se liant à un récepteur de la surface cellulaire, d'un anticorps contre les structures de surface des cellules, d'une structure se liant à un anticorps ou d'un épitope, ainsi que de ligands se liant aux molécules de surface par leur charge, le type de composition caractéristique en acides aminés, et / ou leur glycolysation et / ou leur phosphorylation spécifiques.

10. Protéine de structure selon l'une des revendications 1 à 9, **caractérisée en ce qu'**elle est sélectionnée parmi le VP1 muté, le VP2 muté et / ou VP3 muté.

11. Protéine de structure selon l'une des revendications 1 à 10 sous forme d'une particule d'AAV, en particulier sous la forme d'une capside d'AAV.

12. Acide nucléique codant une protéine de structure selon l'une des revendications 1 à 11.

13. Cellule isolée hormis d'une cellule souche humaine embryonnaire contenant un acide nucléique selon la revendication 12.

14. Procédé de fabrication d'une protéine de structure selon l'une des revendications 1 à 11, **caractérisée en ce qu'**une cellule selon la revendication 13 est cultivée et que le cas échéant, on isole la protéine de structure exprimée.

15. Médicament contenant une protéine de structure selon l'une des revendications 1 à 11, un acide nucléique selon la revendication 12, et / ou une cellule selon la revendication 13.

16. Agent diagnostique contenant une protéine de structure selon l'une des revendications 1 à 11, un acide nucléique selon la revendication 12, et / ou une cellule selon la revendication 13.

17. Utilisation in vitro d'une protéine de structure selon l'une des revendications 1 à 11 pour la modification du tropisme de l'AAV, pour la transformation d'une cellule, pour le diagnostic, pour les tests d'efficacité et / ou pour le ciblage génomique.
